(19)

**Europäisches Patentamt**
**European Patent Office**
**Office européen des brevets**

(11) **EP 3 388 085 B1**

(12) **EUROPEAN PATENT SPECIFICATION**

(45) Date of publication and mention
of the grant of the patent:
**16.12.2020 Bulletin 2020/51**

(21) Application number: **16856629.7**

(22) Date of filing: **13.06.2016**

(51) Int Cl.:
*A61K 48/00* (2006.01)          *A61K 47/42* (2017.01)
*A61P 35/00* (2006.01)

(86) International application number:
**PCT/CN2016/085605**

(87) International publication number:
**WO 2017/067188 (27.04.2017 Gazette 2017/17)**

(54) **PHARMACEUTICAL COMPOSITION AND APPLICATIONS THEREOF**

PHARMAZEUTISCHE ZUSAMMENSETZUNG UND ANWENDUNGEN DAVON

COMPOSITION PHARMACEUTIQUE ET SES UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.10.2015 CN 201510685343**

(43) Date of publication of application:
**17.10.2018 Bulletin 2018/42**

(73) Proprietor: **Sirnaomics, Inc**
**Suzhou, Jiangsu 215123 (CN)**

(72) Inventors:
• **LU, Alan Y**
**Suzhou**
**Jiangsu 215123 (CN)**
• **QIN, Huanlong**
**Suzhou**
**Jiangsu 215123 (CN)**
• **LIU, Menghan**
**Suzhou**
**Jiangsu 215123 (CN)**
• **LU, Patrick Y**
**Suzhou**
**Jiangsu 215123 (CN)**

(74) Representative: **Pawlyn, Anthony Neil**
**Urquhart-Dykes & Lord LLP**
**7th Floor, Churchill House**
**Churchill Way**
**Cardiff CF10 2HH (GB)**

(56) References cited:
**WO-A1-2014/203189          WO-A1-2018/175323
CN-A- 102 573 856          CN-A- 102 776 193
CN-A- 103 007 291          CN-A- 103 623 425**

• **D. PRAMANIK ET AL: "Restitution of Tumor
Suppressor MicroRNAs Using a Systemic
Nanovector Inhibits Pancreatic Cancer Growth in
Mice", MOLECULAR CANCER THERAPEUTICS,
vol. 10, no. 8, 27 May 2011 (2011-05-27), pages
1470-1480, XP055609249, US ISSN: 1535-7163,
DOI: 10.1158/1535-7163.MCT-11-0152**
• **SATISH RAMALINGAM ET AL: "Manipulating
MiRNA Expression: a Novel Approach for Colon
Cancer Prevention and Chemotherapy",
CURRENT PHARMACOLOGY REPORTS, vol. 1,
no. 3, 30 January 2015 (2015-01-30), pages
141-153, XP055609251, DOI:
10.1007/s40495-015-0020-3**
• **ÇIGIR BIRAY AVCI ET AL: "Design of
polyethylene glycol-polyethylenimine
nanocomplexes as non-viral carriers: mir-150
delivery to chronic myeloid leukemia cells :
PEG-PEI nanocomplexes for miRNA delivery",
CELL BIOLOGY INTERNATIONAL., 1 August 2013
(2013-08-01), pages n/a-n/a, XP055609247, GB
ISSN: 1065-6995, DOI: 10.1002/cbin.10157**

## Description

TECHNICAL FIELD

The invention relates to pharmaceutical field, specifically to a pharmaceutical composition and use thereof.

BACKGROUND ART

Colorectal cancer is one of the most common malignant tumors, with incidence listed in the second in developed countries including European and USA, and the third worldwide. Along with continuous improvement of living standard in China and the westernization of diet, the incidence of colorectal cancer, increasing year by year in recent years, has risen to 3-5th among various cancers, especially in big cities. At present, the treatment of colorectal cancer applied drugs with only general effect on tumor but lacked direct targeting colorectal cancer. This situation needs to be improved as soon as possible.

miR-150 inhibits colorectal cancer cell proliferation

The key molecule miR-150 was validated through screening from colorectal cancer progresses at different stages (normal, adenomas, adenocarcinomas) by using miRNA microarrays. It was found in large-scale clinical samples analysis that colorectal cancer patients with low miR-150 expression always had shorter survival and with poorer postoperative chemotherapy sensitivity; however, patients with high miR-150 expression survived longer and with postoperative chemotherapy sensitivity. According to the sequences of miRNAs, four oligos: miR-150 mimic, inhibitor and the controls (mimics control, inhibitor control) were acquired (Table 1), which can be obtained commercially. We then developed a new formulation by applying histidine-lysine Polymer (HKP) as carriers to form nanoparticle formulation. This formulation could be applied as a new generation of targeted therapeutic drug, which inhibited the growth of colon cancer by inducing programmed death of cancer cell in a tumor xerographic mouse models. In conclusion, the chemical synthesis and modification of microRNA drug provides a brand-new therapeutic method for colon cancer, which is different from traditional small molecule or monoclonal antibody drugs. This drug modality shows clear mechanism of action, accurate targets, as well as unique advantages in safety, since it is derived from human body.

Table 1

| Name | Sequence (5'-3') |
|---|---|
| miR-150 mimics | UCUCCCAACCCUUGUACCAGUG |
| miR-150 inhibitor | CACUGGUACAAGGGUUGGGAGA |
| mimics control | UUCUCCGAACGUGUCACGUTT |
| inhibitor control | CAGUACUUUUGUGUAGUACAA |

Regulation of the target gene expression by synthesized miRNAs

MicroRNAs (miRNAs) are endogenous non-coding RNA in eukaryotes which can control protein expression by regulating the degradation of the target messenger RNA. The miRNA mimics or antagomirs could be obtained by chemical synthesis; Synthesized mimics can simulate endogenous maturity miRNAs with high expression, whereas synthesized antagomirs can specifically binding to target miRNAs and weaken the gene silencing effects of endogenous miRNAs, resulted with regulation of the expression of target proteins.

A branched histidine-lysine peptide (HKP) for siRNA delivery *in vivo*

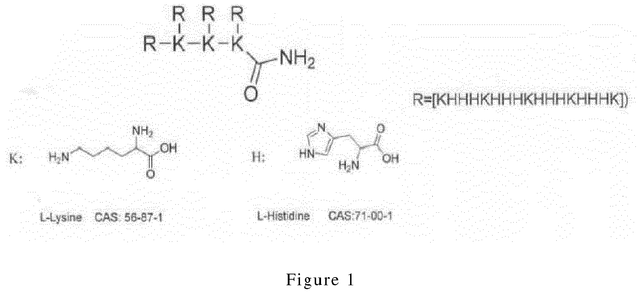

Branchedhistidine-lysine peptide (HKP, Histidine-Lysine Co-polymer), a branched polymer with positive charge (Figure 1), has been successfully used to deliver plasmids and siRNAs *in vivo.* We have carried out the delivery of siRNAs in a variety of tissues, including skin scar, liver, lung, tumor, eye and brain. However, because of the difference in the characteristics between siRNAs and miRNAs, the possibility of miRNAs delivery by using HKPs still needs to be studied.

CN103007291 and CN103623425 both provide for branched carriers in the context of various therapies, including cancer therapies, by do not provide the miRNA's of claim 1.

D Pramanik et el: "Restitution of Tumor Suppressor MicroRNAs Using a Systemic Nanovector Inhibits Pancreatic Cancer Growth in Mice", Molecular Cancer Therapeutics, vol 10, no. 8, 27 May 2011 [pages 1470-1480] provides colon cancer therapy using miRNA143 but absent a carrier of the type claimed in claim 1,

## SUMMARY OF THE INVENTION

In accordance with the present invention, a pharmaceutical composition is provided, which is a targeted drug for the treatment of colorectal cancer.

According to a first aspect of the invention, there is provided a pharmaceutical composition wherein the pharmaceutical composition comprises microRNAs with or without chemical modification, and a carrier that is suitable for delivery *in vivo,* the carrier is a branched histidine-lysine polypeptide or modified compound thereof, characterized in that the micro RNAs include one or more of a miR-150, a miR-143 and a miR-195, the sequence of the miR-150 is 5'-UCUCCCAACCCUUGUACCAGUG-3', the sequence of the miR-143 is 5'-UGAGAUGAAGCACUGUAGCUC-3', the sequence of the miR-195 is 5'-UAGCAGCACAGAAAUAUUGGC-3'. The expression of miR-150 is closely related with prognosis of colorectal cancer by large-scale clinical samples analysis and cellular studies.

Specifically, the miR-150 is a miR-150 analogue constructed according to known sequence of micro RNA *in vitro.*

Specifically, the microRNA is single strand, which is easily degraded, so the chemical modification is used to improve its stability.

Specifically, the chemical modification is formed on a pentose of a single nucleotide or multi-nucleotides of the microRNAs.

Specifically, a chemical group of the chemical modification is fluoro or methoxy.

More Specifically, the chemical modification is formed on 2'-OH in all bases of a single-stranded of the micro-RNAs.

More Specifically, the miR-143 is a miR-143 analogue constructed according to known sequence of micro RNA *in vitro.*

More Specifically, the miR-195 is a miR-195 analogue constructed according to known sequence of micro RNA *in vitro.*

Specifically, the microRNAs include a miR-150 and a miR-143, and the miR-150 and the miR-143 are mixed to form a double-target microRNAs inhibitor to enhance the anti-tumor effect.

Specifically, the microRNAs include a miR-150 and a miR-195, and the miR-150 and the miR-195 are mixed to form a double-target microRNAs inhibitor to enhance the anti-tumor effect.

Specifically, the microRNAs include a miR-143 and a miR-195, and the miR-143 and the miR-195 are mixed to form a double-target microRNAs inhibitor to enhance the anti-tumor effect.

Specifically, the microRNAs include a miR-150, a miR-143 and a miR-195, and the miR-150, the miR-143 and the miR-195 are mixed to form a triple-target microRNAs inhibitor to enhance the anti-tumor effect.

Specifically, the branched histidine-lysine polypeptide as claimed is a positively charged histidine-lysinepolymer (HKP), which is used for nucleic acid delivery in a variety of tissue types.

Specifically, the modified compound of branched histidine-lysine polypeptide is a branched histidine-lysine polymer with a histidine in every branch (HKP+H), which is used for nucleic acid delivery in a variety of tissues with low immune and inflammatory reaction.

Specifically, the branched histidine-lysine polypeptide is H3K4b, which is constituted by three lysine cores and four branches that contain a large number of repetitive histidine and lysine, whose structure is shown in Fig.1.

Specifically, the modified compound of the branched histidine-lysine polypeptide is H3K(+H)4b, with a histidine in each branch of H3K4b. The structure of H3K(+H)4b is the structure shown in Fig. 1 wherein side chain R is replaced with R=KHHHKHHHKHHHHKHHHK.

Specifically, the nitrogen and phosphorus mass ratio (N/P) of the carriers and the microRNAs is between 8:1 and 1:8.

Preferably, the nitrogen and phosphorus mass ratio (N/P) of the carriers and the microRNAs is no less than 4:1.

Specifically, the carrier is a three-component system of RGD-PEG-HKP. RGD and HKP are coupled on both ends of PEG, and the RGD is a polypeptide composed of 7-12 amino acids with specific recognition and adhesion effects on new blood vessels endothelial cells, which can be used as a targeting molecule of micro nucleic acid delivery system.

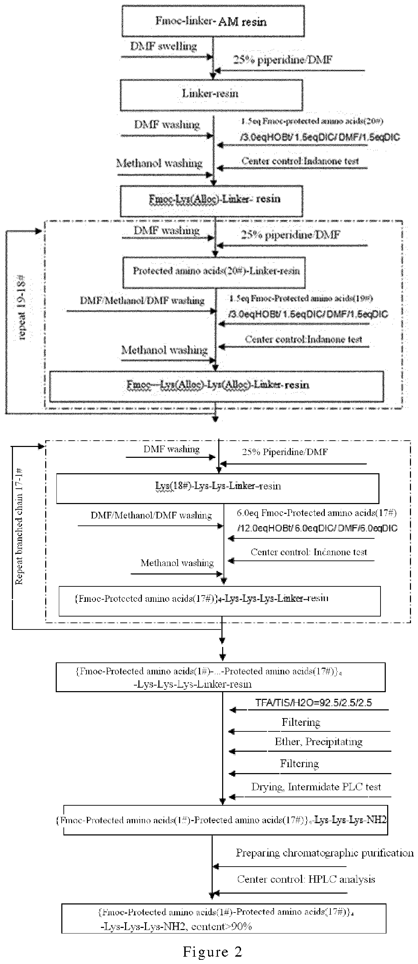

A HKP-end of the three-component system of RGD-PEG-HKP is capable of combining a micro nucleic acid to form a nanoparticle (approximately 150 nm), and a RGD-end on the surface of nanoparticle is capable of targeting tumor cells. Specifically, a molar concentration of the microRNAs is no less than 1 nM. HKP of the present invention is synthesized from an outsourced company, which made the peptide according to the techniques and processes of the inventors. Figure 2 showed the details of HKP synthetic steps.

The pharmaceutical composition for use in colorectal cancer treatment is described herein.

The pharmaceutical composition synergized with small molecule chemical drug or monoclonal antibody for use in colorectal cancer treatment is described herein.

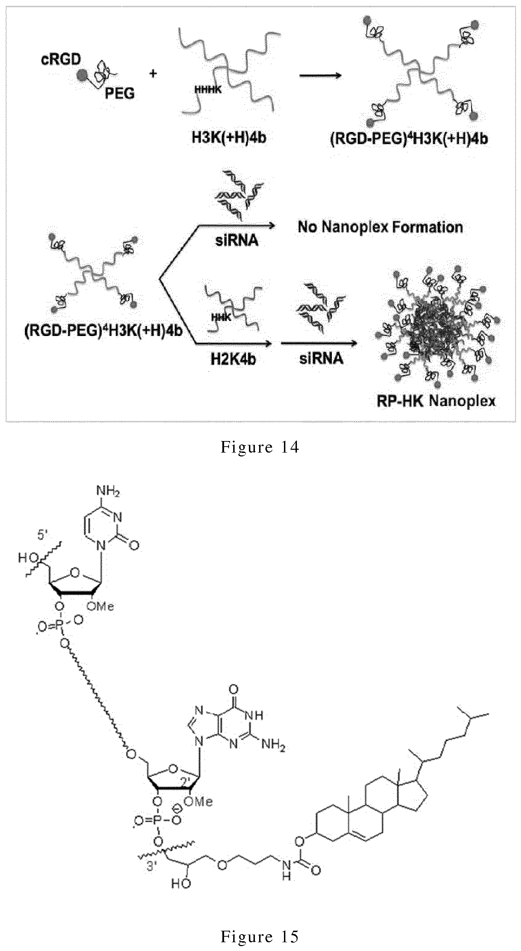

The syntheses of the four single stranded miRNAs in the present invention are synthesized by monomer nucleotides via chemical methods, and chemical modification were introduced to increase their stability. The chemical modification is formed on 2'OH in all the bases of single-stranded miRNAs and the chemical group is methoxy(2'-Ome) or fluoro (2'-F)(Figure 14). Finally, in order to confirm which chemical modification was more beneficial, the miR-150 relative expression of each group was evaluated and compared *in vitro*.

Due to the implementation of the technical scheme above, the invention has the following advantages compared with the current technology:

nanoparticles with different sizes are formed after mixed microRNAs with carrier at different N/P ratios, which are suitable for microRNAs delivery and can inhibit the growth of colon cancer by inducing programmed death of cancer cell, so as to depress the growth of colorectal cancer.

BRIEF DESCRIPTION OF THE DRAWINGS

Figure 1 shows structure diagrams of a branched histidine-lysine polypeptide and a histidine added in each branch thereof. The R stands for amino acid sequence of four branched side chains.
Figure 2 shows synthetic steps of HKP.
Figure 3 shows capacity for entrapping microRNA of HKP with different mass analyzed by agarose gel electrophoresis, wherein, HKP and miRNA (1 ug) was mixed in different mass ratio: 1:1, 2:1, 4:1, 6:1, 8:1, M means DNA Marker.
Figure 4 shows capacity for entrapping microRNA of HKP with different mass analyzed by agarose gel electrophoresis. Wherein, HKP and miRNA (1 ug) was mixed in different mass ratio: 2:1, 2.5:1, 3:1, 3.5:1, 4:1, M means DNA Marker.
Figure 5 shows a standard curve between Ribo Green fluorescent intensity constructed with various miRNA concentrations.
Figure 6 shows the percentage of free-miRNAs to starting total miRNAs in different N/P ratio of HKP and miR-150, namely the entrapment efficiency of HKPs to miRNAs at the different ratios.
Figure 7 shows the results of RT-PCR to confirm the effects of different miRNAs (including mimics, inhibitor, mimics control, inhibitor control) concentrations on miR-150 relative expression *in vitro*; B1K + HKP was serum-free medium + HKP; HKP:miRNA was in the N/P ratio of 4:1; the volume of lipo2000 (1:100, v/v) was consistent in all groups using lipo. The data is expressed as Mean ± Standard Deviation.
Figure 8 shows the effect of different miRNAs (including mimics, inhibitor, mimics control, inhibitor control) concentrations on miR-150 relative expression *in vitro*, with or without chemical modification; the volume of lipo2000 (1:100, v/v) was consistent in all groups using lipo. The data is expressed as Mean±Standard Deviation. M: mimics, MNC: mimics control, I: inhibitor, INC: inhibitor control, *P<0.05, **P<0.01.
Figure 9 shows the tumor inhibitory effect evaluation after nine times treatment with miR-mimics and other control formulation groups that were injected into the tumor tissue. Tumor was formed through subcutaneously inoculated LoVo cells into nude mice. Tumor volume differences between mimics group and other control groups have statistical significant. Wherein, *p < 0.05.
Figure 10 shows the growth trend of tumor volume in each group after repeated treatment.
Figure 11 shows the expression of miR-150 in tumor tissue, wherein *p < 0.05.
Figure 12 is a statistical graph of inhibition of cell division (PCNA) induced by miR-150/HKP drug formulation, wherein *p < 0.05.
Figure 13 is a statistical graph of programmed tumor cell death (TUNEL) induced by miR-150/HKP formulation, wherein *p < 0.05.
Figure 14 shows the preparation process of RGD-HKP/miRNA nanoparticle formulation.
Figure 15 shows the diagram of 2'methoxy modification(2'-Ome).

DESCRIPTION OF THE PREFERRED EMBODIMENTS

The present invention will be further described in detail with reference to specific embodiments, but the present invention is not limited to the following embodiments.

Embodiment 1. Agarose gel electrophoresis analysis of the optimal nitrogen-phosphorus ratio (N/P) between HKP and miR-150.

Agarose gel electrophoresis is an electrophoresis method with the supporter of agarose, which plays a dual role in "molecular sieves" and "electrophoresis". The separation of charged particles not only depends on the character and amount of net charge, but also on the molecular size. Generally, electrophoresis with the supporter of 1% agarose always be used to isolated nucleic acid including in this study, to observe the HKP entrapment capacity to miRNA by 1% agarose gel electrophoresis.

HKP is obtained from two different resources, which was synthesized according to the technology and process of the invention, and mixed with M, respectively in different mass ratio of 1:1, 2:1, 4:1, 6:1, 8:1, to conduct the pre-screening test of nitrogen and phosphorus suitable mass ratio.

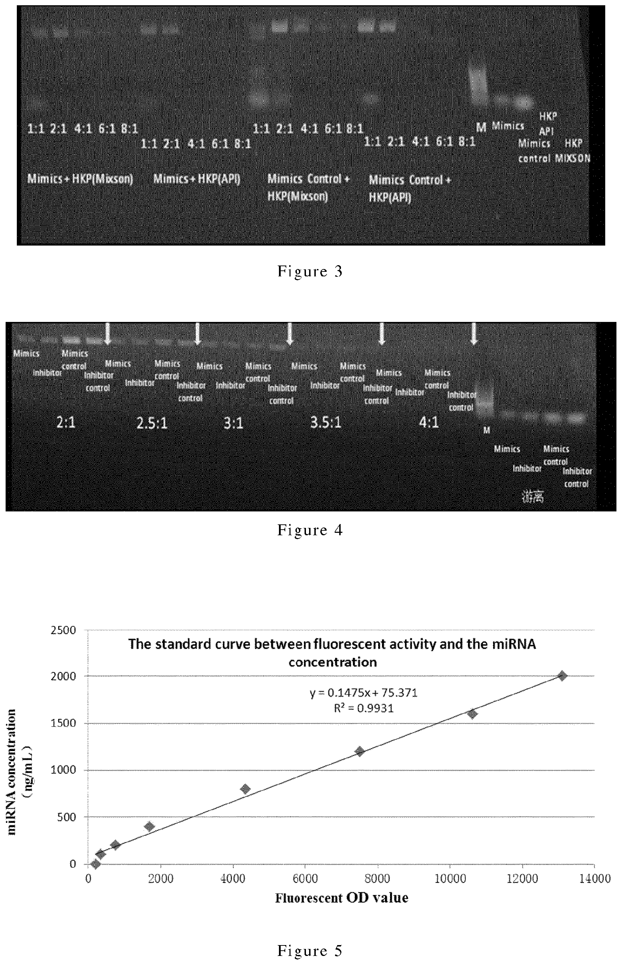

After agarose gel electrophoresis (Figure 2), it is clearly that no free miRNAs in the mass ratio of 4:1, that is, when miRNAs and HKPs are mixed in this or higher ratio, miRNAs can be entrapped completely by HKP. However, there is no free miRNAs in N/P ratio of 2:1. Therefore, in consideration of the cost, five different ratios: 2:1, 2.5:1, 3:1, 3.5:1, 4:1 were further applied to find detailed ratio. According to the results, it's obvious that the HKP from API has better entrapment effect. Therefore, the API's HKP was mixed with four oligos (mimics, inhibitor, mimics control, inhibitor control, respectively) for optimization sequences. However, due to the entrapment effect of HKP (API) with the ratio of 3.5:1 (Figure 3) is not very good in terms of entrapment efficiency and can still detect relatively exposed miRNAs in the well. Therefore, the ratio of 4:1 is much better.

Embodiment 2. Particle size and potential measurements to validate the proper nitrogen to phosphorus mass ratio (N/P) of HKPs and miR-150s.

Dynamic light scattering (DLS) particle size measurement has become a conventional characterization method of nanotechnology because of its accuracy, rapidity and repeatability. Particle size distribution of nanoparticles in aqueous suspension can be measured directly by laser granulometer, which using dynamic light scattering principle.

Zeta potential is also called the electric potential ($\zeta$- potential), it refers to the potential of the shear plane, and is an important indicator of nanoparticle solution dispersion stability. It is the potential difference between continuous phase and the fluid layer with stable stratification that attached to dispersed particles, and can be measured directly by electro dynamics. Electric potential of nanoparticles in aqueous suspension can be measured directly by Zeta Potential Meter, which using electrophoretic light scattering principle.

Mixed HKP (API) with two oligos (mimics, inhibitor,respectively) in the five mass ratios: 2:1, 2.5:1, 3:1, 3.5:1, 4:1, to measure the particle size and potential of the formed nanoparticles. The data of all ratios (Table 2 and Table 3) showed stable potentials and particle sizes.

Table 2

| Mimics | Partical size (nm) | Potential (mV) | Mimics | Partical size (nm) | Potentia (mV) | Mimics | Partical size ( nm ) | Potentia (mV) | Mimics | Partical size (nm) | Potential (mV) | Mimics | Partical size (nm) | Potentia (mV) |
|---|---|---|---|---|---|---|---|---|---|---|---|---|---|---|
| **2:1** | 100.3 | 32 | **2.5:1** | 97.33 | 39.4 | **3:1** | 97.33 | 39.4 | **3.5:1** | 100.2 | 34.8 | **4:1** | 145.9 | 37.2 |
| | 98.82 | 42.8 | | 97.4 | 31.6 | | 97.4 | 31.6 | | 97.68 | 32.7 | | 145.1 | 35.7 |
| | 100 | 34.5 | | 97.01 | 30.6 | | 97.01 | 30.6 | | 99.29 | 29.4 | | 143.9 | 39.4 |
| **Mean** | 99.71 | 36.43 | **Mean** | 97.25 | 33.87 | **Mean** | 97.25 | 33.87 | **Mean** | 99.06 | 32.30 | **Mean** | 144.97 | 37.43 |
| **SD** | 0.78 | 5.65 | **SD** | 0.21 | 4.82 | **SD** | 0.21 | 4.82 | **SD** | 1.28 | 2.72 | **SD** | 1.01 | 1.86 |

Table 3

| Inhibitor | Partical size (nm) | Potential (mV) | Inhibitor | Partical size (nm) | Potentia (mV) | Inhibitor | Partical size (nm) | Potentia (mV) | Inhibitor | Partical size (nm) | Potential (mV) | Inhibitor | Partical size (nm) | Potentia (mV) |
|---|---|---|---|---|---|---|---|---|---|---|---|---|---|---|
| 2:1 | 101 | 30.8 | 2.5:1 | 101.6 | 29.7 | 3:1 | 114 | 35.3 | 3.5:1 | 118.4 | 34.2 | 4:1 | 110.7 | 29.2 |
|  | 102.1 | 31.8 |  | 97.36 | 26.9 |  | 111.3 | 38.7 |  | 114.1 | 29.7 |  | 110.3 | 29.2 |
|  | 100.5 | 25.4 |  | 97.06 | 29.3 |  | 111.8 | 29.7 |  | 115 | 29.1 |  | 108.8 | 25.8 |
| Mean | 101.20 | 29.33 | Mean | 98.67 | 28.63 | Mean | 112.37 | 34.57 | Mean | 115.83 | 31.00 | Mean | 109.93 | 28.07 |
| SD | 0.82 | 3.44 | SD | 2.54 | 1.51 | SD | 1.44 | 4.54 | SD | 2.27 | 2.79 | SD | 1.00 | 1.96 |

Embodiment 3. Analysis of suitable nitrogen and phosphorus mass ratio (N/P) between HKP and miR-150 by entrapment efficiency measurement

RiboGreen RNA quantitation reagent is an ultra-sensitive nucleic acid fluorescent dyes, and RNA quantification will become simple and quick by using this reagent.

RiboGreen fluorescent dye almost has no fluorescence activity in solution; But when it combines with RNA, its fluorescence activity will increase 1000-folds. The fluorescence excitation and emission wavelength of RiboGreen-RNA complex is 500nm and 525nm respectively.

When the RibGreen fluorescent dye is added to the solution, the free miRNA will bind with it and form a RiboGreen-RNAs complex with fluorescent activity. However, HKP-encapsulated miRNAs do not bind to RibGreen fluorescent dyes and do not produce fluorescent activity. Therefore, a standard curve is drawn by using the miRNA fluorescence intensity and the corresponding concentration, and then the corresponding concentration is calculated on the standard curve (Figure. 4) according to the fluorescence of the test sample, and the entrapment efficiency is calculated.

$$\mathrm{Entrapment\ Efficiency}=(1\text{-}C_{\mathrm{RNA}}/C_{\mathrm{initial\ concentration}})\times 100\%$$

HKP (API) and two oligos (mimics, inhibitor) were respectively mixed in the five mass ratios, namely 2:1, 2.5:1, 3:1, 3.5:1, 4:1, to measure the entrapment efficiency.

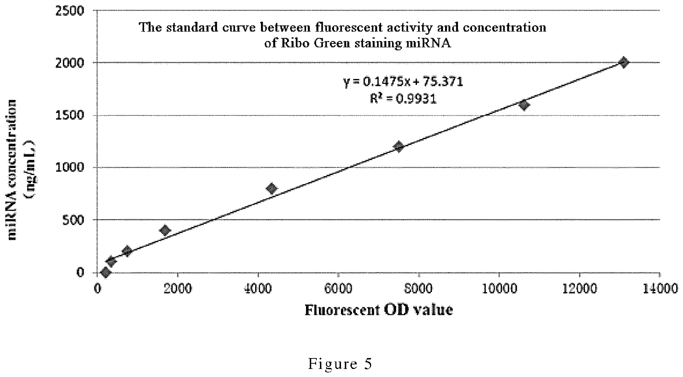

With the increment of HKP (Figure 5), the concentration of free miRNAs was reducing, which means that the entrapment effect was increasing with obvious gradient, and the concentration of free miRNAs was relatively low at the ratio of 4:1, which is suitable for further research.

Embodiment 4. *In vitro* cell biology experiments to analyze the relative activity of miR-150 at the ratio (N/P) of 4:1 (miRNA to HKP)

The complexes of HKP (API) and miRNA (unmodified) (N/P ratio=4:1) were transfected into LOVO cell lines and using real-time fluorescence quantification PCR *in vitro* to detect miR-150 relative expression.

Real-time fluorescence quantitative PCR method: In the real-time fluorescence quantitative PCR reaction system, a fluorescent chemical substance is introduced. As the PCR reaction proceeds, the PCR reaction products are continuously accumulated, and the fluorescence signal intensity also increases in equal proportion. After each cycle, a fluorescence intensity signal is collected, so that changes in the amount of the product can be monitored by changes in the fluorescence intensity, thereby enabling quantitative and qualitative analysis of the starting template.

LOVO cell line was used in the invention. After the cells are transfected for 20-24 hours, the total RNA is extracted from each group of cells, the corresponding cDNA is obtained by reverse transcription PCR, and finally passed through the real-time quantitative PCR to detect the expression of miR-150 in each group of cells. U6 gene was used as a reference gene. Each sample was run in triplicate.

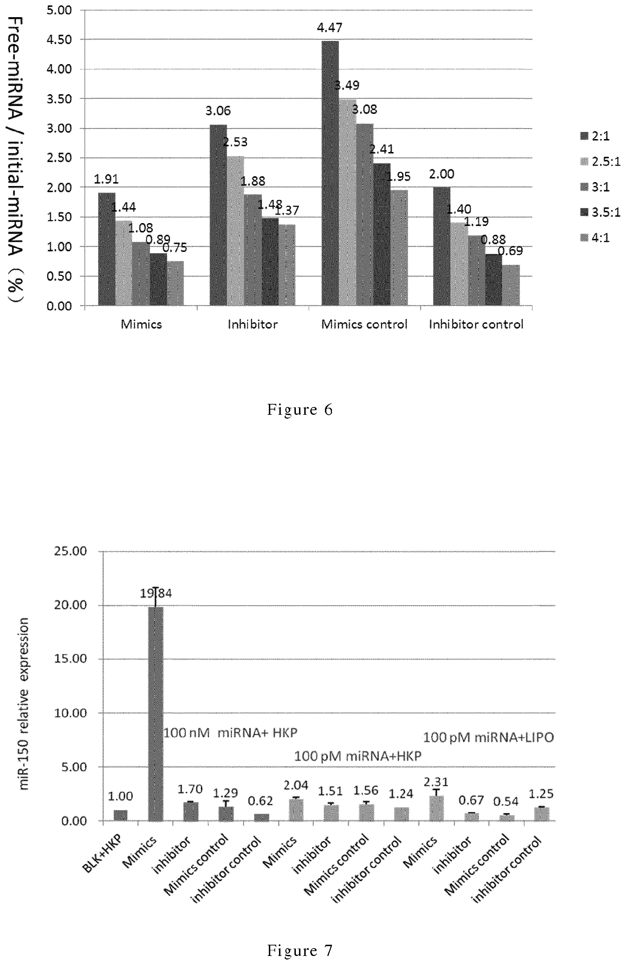

Four oligo miRNAs were detected at a concentration of 100 pM. A group of 100 nM was set up as control. The experimental results showed that the mimics of the three groups had certain effects, especially in 100 nM group (figure 6), indicated that it is appropriate and effective when N/P ratio is 4:1. Therefore, this optimum ratio can be determined as the following formulation preparation ratio.

Embodiment 5. Analysis of miR-mimics (with chemical modification and without) relative expression *in vitro* entrapped by lipo 2000

Nanocomplexes with unmodified mimics and mimics control, as well as chemically modified (2'- and 2'-fluoro modified) mimics and mimics control were encapsulated with lipo 2000 and transfected into LOVO cell lines. The miR-150 relative expression of each group was detected and compared by real-time fluorescent quantitative PCR *in vitro*, in order to confirm whether the chemical modification was beneficial or not.

The most effective one is 2'methoxyl (2'-Ome) modified single stranded miRNA, which could cause a substantial increase of miR-150 intracellular relative expression (Figure 7).

Embodiment 6. Pharmacodynamic study of miR-150 targeted polypeptide formulation

LoVo cells were inoculated subcutaneously in nude mice to form tumors. HKP-encapsulated miR-150 and other control groups of micro nucleic acid drug preparations were injected into the tumor tissue for 9 consecutive treatments. Subcutaneous xenografts were injected once every two days (injected into the tumor tissue at a level of 0.5 cm along the dorsal surface of the skin, and the needles continued to enter the center of the tumor, intratumoral injection). Each injection was 1 OD/70 uL formulation /once with a total of 9 times administration. The entire process took 18 days from the start of dosing to the final collection. Tests have shown that miR-150 has a significant inhibitory effect on tumors (Figure 8). The statistics of tumor volume in each group are shown in Table 4.

Table 4

| Group | Length (mm) | Width(mm) | Volume(mm$^3$) |
|---|---|---|---|
| | 10.07 | 8.49 | 362.92 |
| | 9.71 | 8.54 | 354.08 |
| mimics | 12.03 | 10.06 | 608.73 |
| | 11.78 | 10.36 | 632.17 |
| | 10.03 | 7.61 | 290.42 |
| | 9.76 | 7.64 | 284.84 |
| Mean | | | 422.19 $\pm$ 157.41 |
| mimics control | 13.95 | 11.88 | 984.41 |
| | 15.02 | 13.21 | 1310.52 |
| | 12.49 | 11.01 | 757.01 |
| | 16.44 | 11.37 | 1062.65 |
| | 15.22 | 12.60 | 1208.16 |
| | | | |
| Mean | | | 1064.6 $\pm$ 213.27 |
| inhibitor | 14.31 | 13.06 | 1220.38 |
| | 14.51 | 13.87 | 1395.69 |
| | 15.97 | 12.42 | 1231.73 |
| | 15.88 | 14.92 | 1767.49 |
| | 15.41 | 13.56 | 1416.74 |
| Mean | | | 1406.41 $\pm$ 221.18 |
| inhibitor control | 14.07 | 12.91 | 1172.51 |
| | 10.11 | 9.96 | 501.46 |
| | 12.43 | 11.21 | 781.00 |
| | 14.32 | 12.87 | 1185.96 |
| | 13.25 | 11.73 | 911.55 |
| | 11.99 | 11.36 | 773.65 |
| Mean | | | 887.69 $\pm$ 262.41 |
| transfection | 16.24 | 11.15 | 1009.49 |
| | 13.72 | 11.03 | 834.59 |
| | 15.71 | 15.07 | 1783.90 |
| | 16.17 | 14.68 | 1742.33 |
| | 12.23 | 11.56 | 817.16 |
| | 13.72 | 10.74 | 791.28 |
| Mean | | | 1163.13 $\pm$ 471.17 |

(continued)

| Group | Length (mm) | Width(mm) | Volume(mm$^3$) |
|---|---|---|---|
| blank | 14.50 | 11.99 | 1042.26 |
|  | 15.02 | 12.76 | 1222.76 |
|  | 16.87 | 15.55 | 2039.60 |
|  | 12.59 | 12.01 | 907.99 |
|  | 15.91 | 12.34 | 1211.35 |
| Mean |  |  | 1284.79 ± 441.48 |

Embodiment 7. Changes of tumor volume in the process of targeted therapy

At the beginning of the treatment, the tumor volumes are all approximately 145 mm$^3$. During the course of treatment, except for the miR-150 treatment group, whose tumor growth was significantly slowed, the other groups showed similar growth trends as the untreated control group. From the seventh treatment, tumor growth was significantly inhibited (Figure 9). Statistical data are shown in Table 5.

Table 5

|  | mimics | mimics control | inhibitor | inhibitor control | transfection | blank |
|---|---|---|---|---|---|---|
| 1 | 148.62 | 133.52 | 143.25 | 148.7 | 132.85 | 135.31 |
| 2 | 150.65 | 153.08 | 163.49 | 156.62 | 169.6 | 180.61 |
| 3 | 169.73 | 191.5 | 229.18 | 185.87 | 212.76 | 252.37 |
| 4 | 174.04 | 199.64 | 290.55 | 205.88 | 268.19 | 333.58 |
| 5 | 214.3 | 269.43 | 316.85 | 297.28 | 440.23 | 412.8 |
| 6 | 253.56 | 355.57 | 587.18 | 459.65 | 543.91 | 539.03 |
| 7 | 354.7 | 453.46 | 737.6 | 583 | 664.05 | 735.98 |
| 8 | 382.13 | 696.43 | 947.24 | 742.09 | 848.22 | 975.03 |
| 9 | 422.2 | 1064.6 | 1406.4 | 877.69 | 1163.1 | 1248.8 |

Embodiment 8. Expression of target molecule miR-150 in tumor tissues

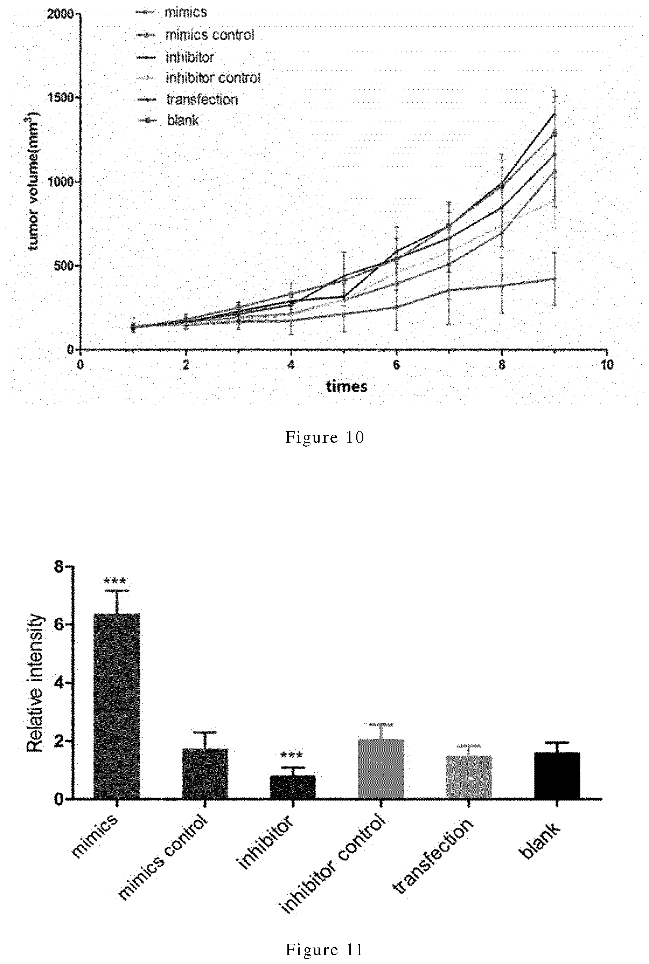

After treatment, animals were treated with euthanasia and tumor tissues were harvested to obtain total RNA. Quantitative RT-PCR was used to detect the expression levels of targeted micro nucleic acids in tumor tissues. It was shown that the expression of micro nucleic acid in miR-150/HKP treatment group was significantly higher than that in other groups (Figure 10), data were shown in Table 6.

Table 6

| Group | 1# | 2# | 3# | Mean |
|---|---|---|---|---|
| mimics | 6.743 | 5.689 | 6.313 | 6.248±0.530 |
| mimics control | 1.766 | 2.244 | 1.558 | 1.856±0.351 |
| inhibitor | 0.980 | 0.807 | 1.053 | 0.943±0.129 |
| inhibitor control | 2.049 | 1.694 | 2.345 | 2.209±0.325 |
| transfection | 1.559 | 1.977 | 1.747 | 1.761±0.209 |
| blank | 2.121 | 1.772 | 1.523 | 1.805±0.301 |

Embodiment 9A. Programmed tumor cell death (PCNA) was induced by miR-150/HKP formulation

The treated tumor tissues were detected and proliferating cell nuclear antigen (PCNA) was evaluated. PCNA in tumor tissues treated with miR-150/HKP drugs was significantly different from other control groups and untreated groups. PCNA was substantially reduced in the tumor cell nucleus after miR-150/ HKP formulation treatment, indicated tumor cell death (Figure 11). The data were shown in table 7.

Table7

| Group | PCNA positive nuclei (%) | | | Mean |
|---|---|---|---|---|
| mimics | 0.090583 | 0.096861 | 0.086996 | $0.0914 \pm 0.00499$ |
| mimics control | 0.323956 | 0.344416 | 0.307758 | $0.3253 \pm 0.01837$ |
| inhibitor | 0.478066 | 0.498657 | 0.457475 | $0.4781 \pm 0.02059$ |
| inhibitor control | 0.366551 | 0.396014 | 0.348354 | $0.3703 \pm 0.02405$ |
| transfection | 0.395541 | 0.423617 | 0.377374 | $0.3988 \pm 0.02329$ |
| blank | 0.391525 | 0.432203 | 0.363559 | $0.3957 \pm 0.03452$ |

Embodiment 9B. Programmed tumor cell death (TUNEL) was induced by miR-150/HKP formulation

After the treatment, it was detected that miR-150/HKP treatment group showed significant difference from other control groups by classic TUNEL method. TUNEL nuclei got a substantial increase in the tumor cell after miR-150/ HKP formulation treatment, indicated the treatment induced programmed tumor cell death (Figure 11). The data were shown in table 8.

Table8

| Group | TUNEL-positive nuclei (%) | | | Mean |
|---|---|---|---|---|
| mimics | 0.2782739 | 0.3007692 | 0.2476548 | $0.2756 \pm 0.02666$ |
| mimics control | 0.1355891 | 0.1039606 | 0.1182176 | $0.1193 \pm 0.01584$ |
| inhibitor | 0.0811801 | 0.09044278 | 0.09593246 | $0.0892 \pm 0.007456$ |
| inhibitor control | 0.1250844 | 0.1397036 | 0.1009606 | $0.1219 \pm 0.01956$ |
| transfection | 0.1332083 | 0.1305797 | 0.1414653 | $0.1351 \pm 0.00568$ |
| blank | 0.100844 | 0.1183321 | 0.0939606 | $0.1044 \pm 0.01256$ |

Embodiment 10. RGD-HKP can target to deliver miRNA to tumor tissue

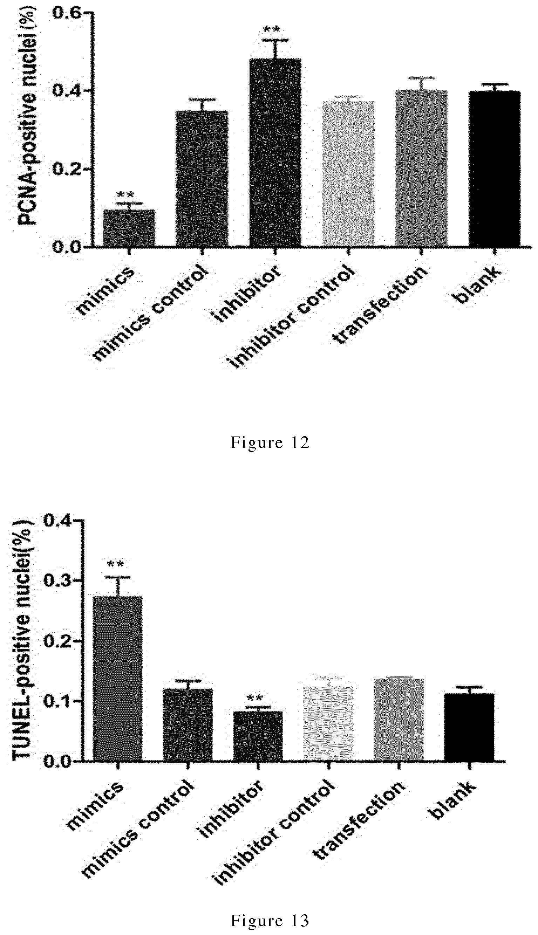

The HKP/siRNA nanoparticles modified by application of cRGD can have affinity for $\alpha$ v$\beta$3 and $\alpha$vA$\beta$5 integrins on the surface of neovascular endothelial cells. Due to the large number of neovascularizations in the tissues near the tumor, and abundant expression of $\alpha$v$\beta$3 and $\alpha$v$\beta$5 integrins, the HKP (cRGD modified)/siRNA nanoparticles we designed could enhance the miRNA enrichment near the tumor tissue and miRNA entered into tumor cells effectively to induce cell death. Figure 13 shows the RGD-HKP/miRNA nanoparticle preparation process.

Embodiment 11. miR-143 for the treatment of colorectal cancer

miR-143 is significantly down-regulated in colorectal cancer tissues, and its expression level can be regulated through different pathways. Studies have shown that PI3K/Akt, MAPK and HGF/MET signal paths were affected by the down-regulation of miR-143. Meanwhile, miR-143 can decrease the expression of Ras, Erk5, Akt and MCC1. If miR-143 is added to rectal cancer cells, apoptosis of cancer cells can be induced and slower tumor growth can be observed in mouse animal experiments. Therefore, the application of miR-143 alone can achieve the treatment of rectal cancer.

Embodiment 12. miR-195 for colorectal cancer treatment to reduce tumor resistance to cytotoxic drugs

Through cytological tests, it was found that knockdown of miR-195 HT29 and LOVO cells significantly inhibited DOX (Doxorubicin) -induced cytotoxicity. At the same time, the direct involvement of miR-195 inhibits the expression of BCL2L2. Further studies have shown that low-profile miR-195 expression can enhance DOX resistance in tumor precursor cells and reduce the rate of tumor cell apoptosis. When miR-195 is overexpressed, tumor cells are more sensitive to DOX and induce apoptosis. These results suggest that the application of miR-195 as a therapeutic method will gradually reduce the resistance of tumor cells to DOX and enhance the therapeutic effect.

Embodiment 13. miR-150/miR143/miR195 miRNAs for the treamtnet of colorectal cancer

Based on the embodiment 11 and 12, we could assume and confirm that the miRNAs cocktail of miR-150/miR143, the miR-150/miR195, the miR143/miR195, or the miR-150/miR143/miR195 trinity would further enhance the treatment effect.

Embodiment 14. Synergized miR-150 with small molecule-based drug for anti-tumor treatment

Stivarga (regorafenib, Tablets Riegefini) is the FDA approved new indication in 2013 for the treatment of advanced gastrointestinal stromal tumors. Stivarga is a kinase inhibitor apply to the chemotherapy that was used based on [fluoropyrimidine]-, [oxaliplatin]- and [irinotecan]- in the past. It is a kind of anti-VEGF treatment including KRAS wild-type, as well as a kind of anti-EGFR-treatment for metastatic colorectal cancer (CRC). The use of miR-150 in combination with this small molecule targeted drug to treat predictable good outcomes in rectal cancer.

Embodiment 15. Synergized miR-150 with monoclonal antibody for anti-tumor treatment

Lots of monoclonal antibody drugs are approved by the FDA as first-line or second-line drugs for colorectal cancer treatment, such as Bevacizumab (Avastin), Betuximab (Erbitux), and Panitumumab (Vectibix). The combination of miR-150 and antibody drugs which have been applied in clinical application would be an approach with a great prospect for the treatment.

## Claims

1. A pharmaceutical composition, wherein the pharmaceutical composition comprises micro RNAs with or without chemical modification, and a carrier suitable for delivery *in vivo*, the carrier is a branched histidine-lysine polypeptide **characterized in that** the micro RNAs include one or more of a miR-150, a miR-143 and a miR-195, the sequence of the miR-150 is 5'-UCUCCCAACCCUUGUACCAGUG-3', the sequence of the miR-143 is 5'-UGAGAUGAAGCACUGUAGCUC-3', the sequence of the miR-195 is 5'-UAGCAGCACAGAAAUAUUGGC-3'.

2. The pharmaceutical composition according to claim 1, **characterized in that**, the micro RNAs include any two of the miR-150, the miR-143 and the miR-195 which are mixed to form a double-target microRNAs inhibitor; or, the micro RNAs include the miR-150, the miR-143 and the miR-195 which are mixed to form a triple-target microRNAs inhibitor.

3. The pharmaceutical composition according to any one of claims 1-2, is **characterized in that**, the micro RNA is single strand.

4. The pharmaceutical composition according to any one of claims 1-3, is **characterized in that**, the chemical modification is formed on a pentose of a single nucleotide or multi-nucleotides of the micro RNAs.

5. The pharmaceutical composition according to any one of claims 1-4, is **characterized in that**, a chemical group of the chemical modification is fluoro or methoxy.

6. The pharmaceutical composition according to claim 5, is **characterized in that**, the chemical modification is formed on 2'OH in all bases of a single stranded of the micro RNAs.

7. The pharmaceutical composition according to any one of claims 1-6, is **characterized in that**, the branched histidine-lysine polypeptide is a positively charged branched histidine-lysine polymer.

8. The pharmaceutical composition according to any one of claims 1-7, is **characterized in that**, the modified compound of the branched histidine-lysine polypeptide is a branched histidine-lysine polymer with a histidine added in each branch thereof.

9. The pharmaceutical composition according to any one of claims 1-8, is **characterized in that**, the branched histidine-lysine polypeptide is H3K4b or H3K(+H)4b.

10. The pharmaceutical composition according to any one of claims 1-9, is **characterized in that**, the nitrogen and phosphorus mass ratio of the carrier and the micro RNAs is between 8:1 and 1:8.

11. The pharmaceutical composition according to any one of claims 1-10, is **characterized in that**, the carrier is a three-component system of RGD-PEG-HKP, wherein RGD and HKP are coupled on both ends of PEG.

12. The pharmaceutical composition according to any one of claims 1-11, is **characterized in that**, a molar concentration of the micro RNAs is no less than 1 nM.

13. The pharmaceutical composition according to any one of claims 1 -12 for use in the treatment of colorectal cancer.

14. The pharmaceutical composition according to any one of claims 1-12 synergized with small molecule chemical drug or monoclonal antibody for use in the treatment of colorectal cancer.


**Patentansprüche**

1. Pharmazeutische Zusammensetzung, wobei die pharmazeutische Zusammensetzung Mikro-RNAs mit oder ohne chemische Modifikation sowie einen Trägerstoff, der für die *In-vivo*-Abgabe geeignet ist, umfasst, wobei der Trägerstoff ein verzweigtes Histidin-Lysin-Polypeptid ist, charakterisiert dadurch, dass die Mikro-RNAs eine oder mehrere von einer miR-150, einer miR-143 und einer miR-195 enthalten, wobei die Sequenz der miR-150 5'-UCUCCCAACCCUUGUACCAGUG-3', die Sequenz der miR-143 5'-UGAGAUGAAGCACUGUAGCUC-3', die Sequenz der miR-195 5'-UAGCAGCACAGAAAUAUUGGC-3' ist.

2. Pharmazeutische Zusammensetzung gemäß Anspruch 1, charakterisiert dadurch, dass die Mikro-RNAs zwei der miR-150, miR-143 und miR-195 enthalten, welche vermischt sind, um einen Doppelziel-Mikro-RNA-Inhibitor zu bilden; oder die Mikro-RNAs die miR-150, die miR-143 und die miR-195 enthalten, welche vermischt sind, um einen Dreifachziel-Mikro-RNA-Inhibitor zu bilden.

3. Pharmazeutische Zusammensetzung gemäß einem der Ansprüche 1 bis 2, charakterisiert dadurch, dass die Mikro-RNA einzelsträngig ist.

4. Pharmazeutische Zusammensetzung gemäß einem der Ansprüche 1 bis 3, charakterisiert dadurch, dass die chemische Modifikation an einer Pentose eines Einzelnukleotids oder von Multinukleotiden der Mikro-RNAs gebildet ist.

5. Pharmazeutische Zusammensetzung gemäß einem der Ansprüche 1 bis 4, charakterisiert dadurch, dass eine chemische Gruppe der chemischen Modifikation Fluor oder Methoxy ist.

6. Pharmazeutische Zusammensetzung gemäß Anspruch 5, charakterisiert dadurch, dass die chemische Modifikation an 2'OH in allen Basen eines Einzelstranges der Mikro-RNAs gebildet ist.

7. Pharmazeutische Zusammensetzung gemäß einem der Ansprüche 1 bis 6, charakterisiert dadurch, dass das verzweigte Histidin-Lysin-Polypeptid ein positiv geladenes verzweigtes Histidin-Lysin-Polymer ist.

8. Pharmazeutische Zusammensetzung gemäß einem der Ansprüche 1 bis 7, charakterisiert dadurch, dass die modifizierte Verbindung des verzweigten Histidin-Lysin-Polypeptids ein verzweigtes Histidin-Lysin-Polymer mit einem Histidin, welches an jeder Verzweigung addiert wurde, ist.

9. Pharmazeutische Zusammensetzung gemäß einem der Ansprüche 1 bis 8, charakterisiert dadurch, dass das verzweigte Histidin-Lysin-Polypeptid H3K4b oder H3K(+H)4b ist.

**10.** Pharmazeutische Zusammensetzung gemäß einem der Ansprüche 1 bis 9, charakterisiert dadurch, dass das Stickstoff- und Phosphor-Massenverhältnis des Trägerstoffes und der Mikro-RNAs zwischen 8:1 und 1:8 liegt.

**11.** Pharmazeutische Zusammensetzung gemäß einem der Ansprüche 1 bis 10, charakterisiert dadurch, dass der Trägerstoff ein Dreikomponentensystem von RGD-PEG-HKP ist, wobei RGD und HKP an beide Enden von PEG gekoppelt sind.

**12.** Pharmazeutische Zusammensetzung gemäß einem der Ansprüche 1 bis 11, charakterisiert dadurch, dass die molaren Konzentrationen der Mikro-RNAs nicht kleiner als 1 nM sind.

**13.** Pharmazeutische Zusammensetzung gemäß einem der Ansprüche 1 bis 12 zur Verwendung bei der Behandlung von kolorektalem Karzinom.

**14.** Pharmazeutische Zusammensetzung gemäß einem der Ansprüche 1 bis 12 in Synergie mit einem niedermolekularen chemischen Medikament oder einem monoklonalen Antikörper zur Verwendung bei der Behandlung von kolorektalem Karzinom.

**Revendications**

**1.** Composition pharmaceutique, dans laquelle la composition pharmaceutique comprend des microARN avec ou sans modification chimique, et un support approprié pour une administration *in vivo*, le support est un polypeptide histidine-lysine ramifié, **caractérisée en ce que** les microARN incluent un ou plusieurs des miR-150, miR-143 et miR-195, la séquence du miR-150 est 5'-UCUCCCAACCCUUGUACCAGUG-3', la séquence du miR-143 est 5'-UGAGAUGAAGCACUGUAGCUC-3', la séquence du miR-195 est 5'-UAGCAGCACAGAAAUAUUGGC-3' .

**2.** Composition pharmaceutique selon la revendication 1, **caractérisée en ce que** les microARN incluent deux quelconques des miR-150, miR-143 et miR-195 qui sont mélangés pour former un inhibiteur de microARN à double cible ; ou, les microARN incluent miR-150, miR-143 et miR-195 qui sont mélangés pour former un inhibiteur de microARN à triple cible.

**3.** Composition pharmaceutique selon l'une quelconque des revendications 1 et 2, **caractérisée en ce que** le microARN est un microARN simple brin.

**4.** Composition pharmaceutique selon l'une quelconque des revendications 1 à 3, **caractérisée en ce que** la modification chimique est formée sur un pentose d'un seul nucléotide ou de multi-nucléotides des microARN.

**5.** Composition pharmaceutique selon l'une quelconque des revendications 1 à 4, **caractérisée en ce qu'**un groupe chimique de la modification chimique est un groupe fluoro ou méthoxy.

**6.** Composition pharmaceutique selon la revendication 5, **caractérisée en ce que** la modification chimique est formée sur 2'OH dans toutes les bases de microARN simple brin.

**7.** Composition pharmaceutique selon l'une quelconque des revendications 1 à 6, **caractérisée en ce que** le polypeptide histidine-lysine ramifié est un polymère histidine-lysine ramifié chargé positivement.

**8.** Composition pharmaceutique selon l'une quelconque des revendications 1 à 7, **caractérisée en ce que** le composé modifié du polypeptide histidine-lysine ramifié est un polymère histidine-lysine ramifié avec une histidine ajoutée dans chaque ramification de celui-ci.

**9.** Composition pharmaceutique selon l'une quelconque des revendications 1 à 8, **caractérisée en ce que** le polypeptide histidine-lysine ramifié est H3K4b ou H3K(+H)4b.

**10.** Composition pharmaceutique selon l'une quelconque des revendications 1 à 9, **caractérisée en ce que** le rapport massique azote sur phosphore du support et des microARN est compris entre 8 : 1 et 1 : 8.

**11.** Composition pharmaceutique selon l'une quelconque des revendications 1 à 10, **caractérisée en ce que** le support est un système à trois composants de RGD-PEG-HKP, où RGD et HKP sont couplés sur les deux extrémités de PEG.

**12.** Composition pharmaceutique selon l'une quelconque des revendications 1 à 11, **caractérisée en ce qu'**une concentration molaire des microARN n'est pas inférieure à 1 nM.

**13.** Composition pharmaceutique selon l'une quelconque des revendications 1 à 12 pour une utilisation dans le traitement du cancer colorectal.

**14.** Composition pharmaceutique selon l'une quelconque des revendications 1 à 12 en synergie avec un anticorps monoclonal ou un médicament chimique à petites molécules pour une utilisation dans le traitement du cancer colorectal.

Figure 1

Figure 2

Figure 3

Figure 4

Figure 5

Figure 6

Figure 7

Figure 5

Figure 10

Figure 11

Figure 12

Figure 13

Figure 14

Figure 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103007291

- CN 103623425

**Non-patent literature cited in the description**

- **D PRAMANIK.** Restitution of Tumor Suppressor MicroRNAs Using a Systemic Nanovector Inhibits Pancreatic Cancer Growth in Mice. *Molecular Cancer Therapeutics,* 27 May 2011, vol. 10 (8), 1470-1480